# EUROPEAN PATENT APPLICATION

(11) **EP 1 454 589 A1**
(43) Date of publication of application: **08.09.2004**
(21) Application number: 04251244.2
(22) Date of filing: 03.03.2004
(51) Int. Cl.: A61B 17/02

(54) **Portal apparatus for use in spinal distraction**

(30) Priority: 03.03.2003 US 451940 P; 15.09.2003 US 663212
(71) Applicant: CENTERPULSE SPINE-TECH, INC., Minneapolis, MN 55439-2029 (US)
(72) Inventor: Dant, Jack A., St. Paul, Minnesota 55105 (US)
(74) Representative: Humphreys, Ceris Anne

(57) **Abstract**

A portal (100) for use in a spinal implantation procedure is disclosed herein. The portal (100) includes base (102) and first and second paddles (104,106) pivotally coupled to the base. The paddles (014, 106) can be pivoted between a distraction orientation and an insertion orientation. In the insertion orientation, the paddles (104, 106) are adapted for insertion between vertebrae that are in need of distraction. In the distraction orientation, the paddles are adapted to hold the vertebrae spaced apart at a distracted spacing. In the distraction orientation, the portal (100) defines a portal window (109) for accessing the disc space between the two distracted vertebrae.

## Description

### Cross Reference To Related Applications

This application claims priority from provisional application Serial No. 60/451,940, filed March 3, 2003, and which is incorporated herein by reference.

### Technical Field

The present invention relates to the field of spinal surgeries, and, in particular, to a method and apparatus of spinal distraction.

### Background

The human spine includes a column of vertebrae separated by intervertebral discs. Spinal surgery is sometimes required to correct various abnormalities and/or conditions involving deterioration of the spine. Many spinal surgical procedures, including spinal fusion, require distraction, or separation of adjacent vertebrae. The distraction facilitates access to the intervertebral space and the disc thereinbetween.

One type of distraction can be referred to as "wedge-style" distraction. "Wedge-style" distraction is performed with one or more wedges inserted into the intervertebral space with or without a mallet. This procedure is accomplished as follows. First, a tip of the wedge is inserted into the disc space. Next, the surgeon advances the wedge into the disc space via manual force and/or the use of a mallet to strike an impaction surface of the wedge. This drives the wedge(s) into the disc space. As the wedge is driven into place, a taper of the wedge forces the vertebrae apart and distracts the vertebrae. Example "wedge-style" distraction systems are disclosed in U.S. Patent Nos. 6,224,599 and 5,772,661, which are hereby incorporated by reference in their entireties.

### Summary

One inventive aspect of the present disclosure relates to a portal including two expansion structures separated by a bridge structure. The expansion structures are moveable relative to the bridge structure between an insertion position and a distraction position. In the distraction position, the expansion structures are adapted to hold two vertebrae in a distracted state, with the bridge structure and the expansion structures cooperating to define a portal for accessing the intervertebral space. In the insertion position, the expansion structures are adapted to be inserted between two non-distracted vertebrae.

Another inventive aspect of the present disclosure relates to a portal having paddles that can be pivoted from a closed position to an open position. When in the closed position, the portal is sized to be inserted into the intervertebral space. When moved to the open position, the portal is adapted to distract two vertebrae, and to provide a portal or window for accessing the intervertebral space.

A further inventive aspect of the present disclosure relates to a method for distracting two adjacent vertebrae with a flip-up portal. The method includes inserting the flip-up portal into the disc space between two adjacent vertebrae while the flip-up portal is in a closed position. The method also includes opening the flip-up portal while the flip-up portal is in the disc space, so that the flip-up portal distracts the adjacent vertebrae and defines a window for accessing the intervertebral space. In one example, the flip-up portal has paddles that are approximately parallel to a base of the flip-up portal when the flip-up portal is in the closed position, and the paddles are approximately perpendicular to the base when the flip-up portal is in the open position.

Examples of a variety of inventive aspects are set forth in the description that follows. It is to be understood that both the forgoing general description and the following detailed description are exemplary and explanatory only and are not restrictive of the broad inventive aspects that underlie the examples disclosed herein.

### Brief Description of the Drawings

Figure 1 is a perspective view of an exemplary flip-up portal in an open position.
Figure 2 is a top view of the portal of Figure 1;
Figure 3 is a front view of the portal of Figure 1;
Figure 4 is a side view of the portal of Figure 1;
Figure 5 is a top view of the portal of Figure 1 in a closed position;
Figure 6 is a front view of the portal of Figure 1 in a closed position;
Figure 7 is a side view of the portal of Figure 1 in a closed position;
Figure 8 is a side view of an alternative portal having a lordotic taper;
Figure 9 is a side view of an alternative portal having side viewing windows to increase the radiopacity of the paddles;
Figure 10 illustrates an actuator/inserter tool adapted for use with the portal of Figure 1;
Figure 11 illustrate the portal of Figure 1 mounted on the actuator of Figure 10;
Figure 12 shows the actuator of Figure 10 being used to insert the portal of Figure 1 into an intervertebral space, the portal is shown in the closed position
Figure 13 shows the actuator of Figure 10 being used to open the portal of Figure 1 within the intervertebral space defined between two vertebrae; and

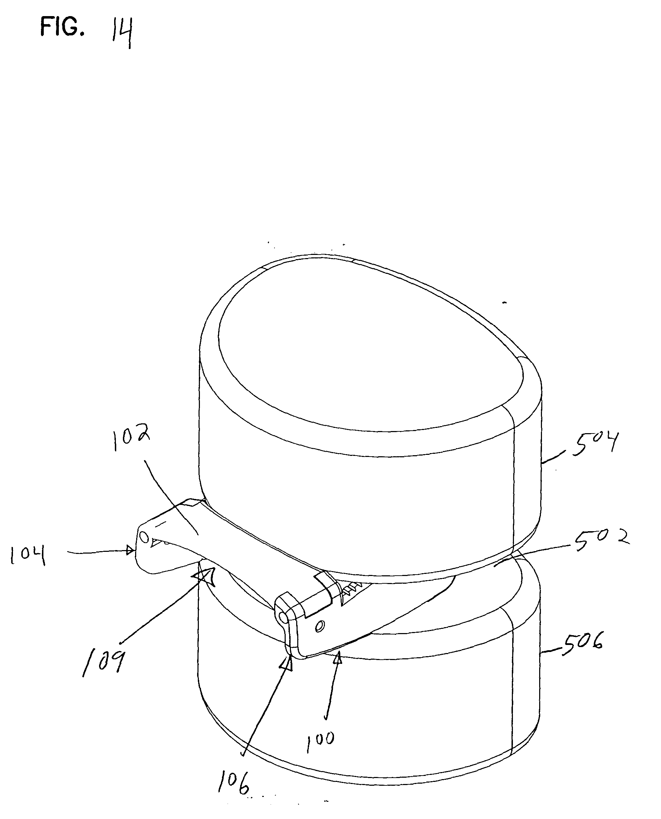

Figure 14 shows the portal of Figure 1 inserted with the intervertebral space after the actuator has been removed.

### Detailed Description

Inventive aspects in accordance with the present disclosure relate to methods and devices for providing spinal distraction. In one embodiment, distraction is provided by a flip-up portal having a base and two paddles that are pivotally coupled to the base. The paddles can be pivoted relative to the base between open and closed positions. In the closed position, the paddles of the flip-up portal are folded inwardly to provide a compact orientation. In the open position, the paddles are pivoted outwardly relative to the base to provide an enlarged orientation. When pivoted from the closed position to the open position, the paddles are moved (e.g., flipped, pivoted or otherwise manipulated) from a less upright position to a more upright position.

In use, the paddles of the portal are inserted into the intervertebral disc space while in the closed position. After insertion into the disc space, the paddles are moved to the open position to distract the two adjacent vertebrae apart, and to provide a portal widow for accessing the disc space. In one non-limiting embodiment, the paddles are generally parallel to the base when in the closed position, and are generally perpendicular to the base when in the open position.

In one example, spinal distraction is accomplished as follows. First, a partial or complete discectomy is performed. Next, a flip-up portal is installed onto an actuator that is configured to insert the flip-up portal into the disc space and the actuator is then used to insert the paddles of the portal into the disc space. Thereafter, the actuator is used to open the flip-up portal to open, thereby distracting the disc space. Thereafter, the actuator is removed while leaving the flip-up portal in the disc space. A surgical procedure can then be performed through the portal. Example surgical procedures include preparation of the vertebral end plates by known techniques such as rasping the cortical end plates and/or chiseling away portions of the vertebral end plates to expose cancellous bone and/or bone tapping. After the end plates have been prepared, one or more vertebral implants can be inserted through the portal into the intervertebral space to stabilize the adjacent vertebral bodies. After the surgical procedure is complete, the flip-up portal is removed from the disc space leaving the implant in the intervertebral space.

It will be appreciated that distraction devices in accordance with the principles of the present disclosure are preferably used for spinal distraction, but could also be used for any other type of bone/tissue distraction.

The present disclosure provides examples of inventive aspects that provide advantages over known distraction devices and methods. Examples of advantages provided by inventive aspects disclosed herein include elimination of misdirected mallet strikes that can occur with wedge style distraction systems, and improved control of the distraction process. Other inventive aspects relate to distraction devices and methods that impose less strain in patient physiology, and are less physically demanding for surgeons.

### I. Example Portals

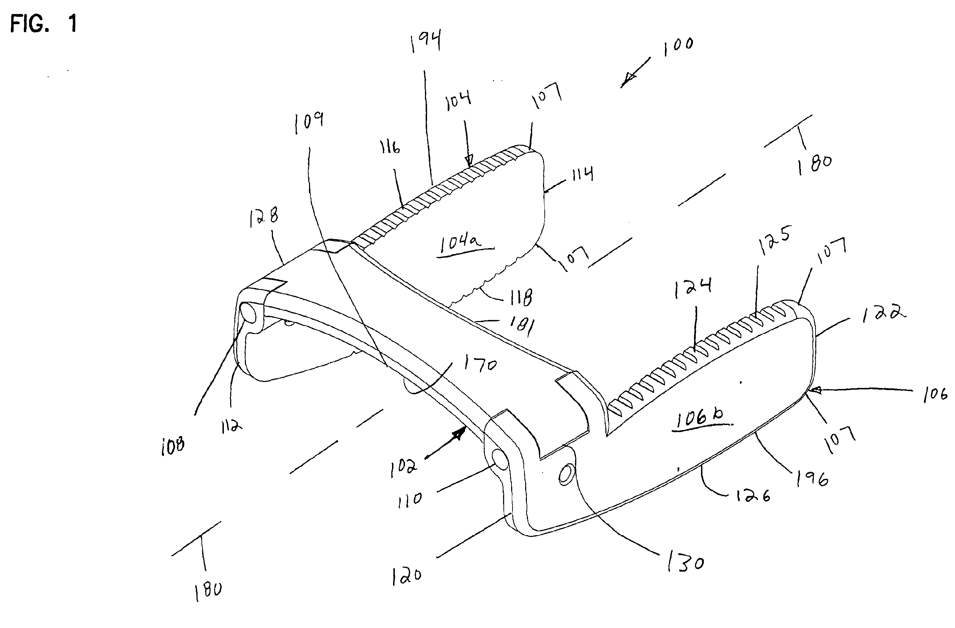

An example flip-up portal 100 in accordance with the present disclosure is shown in Figures 1-7. The flip-up portal 100 includes a base 102 (e.g., a bridge member), paddles 104, 106, and pivot hinges 108,110. Paddles 104 and 106 are sized to fit into the disc space defined between two vertebrae of a human spine. Base 102 has opposite ends 128,130. Paddle 104 is pivotally connected to end 128 of base 102 with pivot hinge 108. Paddle 106 is pivotally connected to end 130 of base 102 with pivot hinge 110.

Paddle 104 has edges 112, 114, 116, 118, and paddle 106 has edges 120, 122, 124, 126. Edges 112 and 114 are approximately parallel, edges 116 and 118 are approximately parallel, edges 120 and 122 are approximately parallel, edges 124 and 126 are approximately parallel, edge 112 is approximately perpendicular to edge 116, and edge 120 is approximately perpendicular to edge 124. In the depicted embodiment, edges 116, 118, 124 and 126 each define a slightly convex curvature.

The paddle 104 has minor sides defined by the edges 112, 114, 116, 118, and major sides 104a, 104b that extend between the edges 112, 114, 116, 118. Similarly, paddle 106 has minor sides defined by edges 120, 122, 124, 126 and major sides 106a, 106b that extend between the edges 120, 122, 124, 126. In the depicted embodiment, the major sides 104a, 104b, 106a, 106b are generally planar. It will be appreciated that the major sides could also be curved.

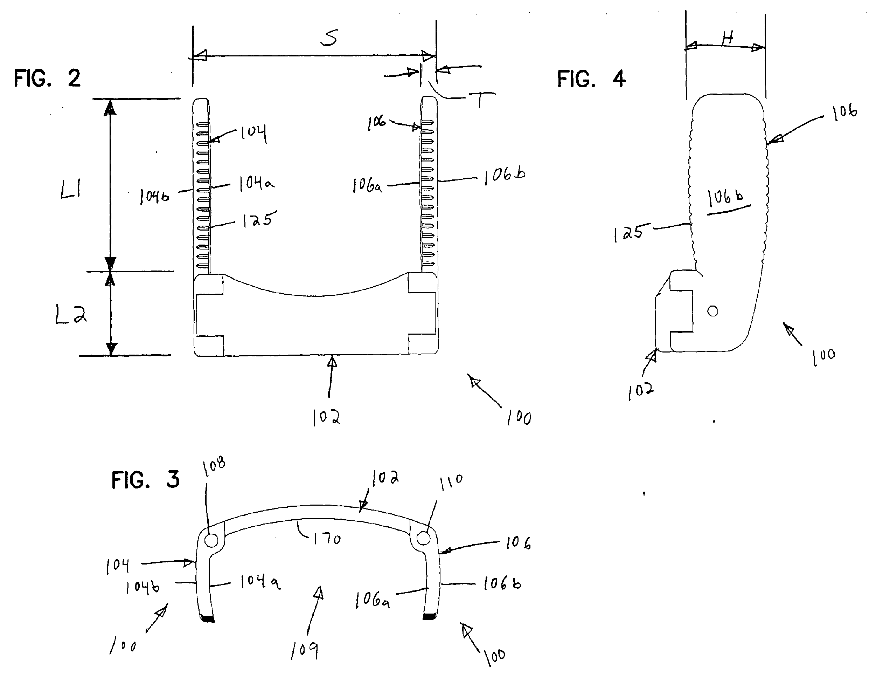

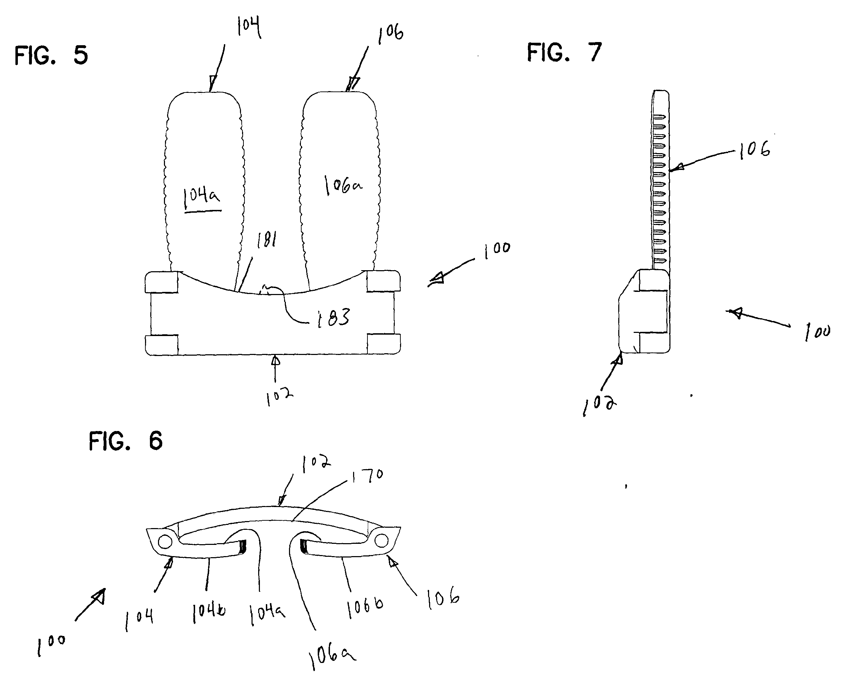

Edges 116 and 118 of paddle 104 and edges 124 and 126 of paddle 106 are configured to prevent sliding when flip-up portal 100 is installed in the disc space. For example, the edges can be serrated, bumped, knurled or otherwise provided with structures for preventing displacement. As shown in Fig. 2 and 4, the edges include serrations 125. Paddles 104 and 106 have rounded comers 107.

The flip-up portal 100 is shown in a closed position in Figures 5-7. In the closed position, the major sides 104a, 106a of the paddles 104,106 have do not oppose one another. In the depicted embodiment, the paddles 104, 106 are generally co-planar and are approximately parallel to the base 102.

Figures 1-4 show the flip-up portal 100 in the open position. In the open position, the major surfaces 104a, 106a of the paddles 104,106 oppose one another, and the major surfaces 104b, 106b face away from one another. In the depicted embodiment, the paddles 104, 106 are approximately perpendicular to the base 102, and approximately parallel to each other. In alternative embodiments, the paddles can be pivoted to open positions that define angles greater than 90 degrees relative to the base 102, or less than 90 degrees relative to the base 102.

Referring to Figure 1, the base 102 and the paddles 104, 106 cooperate to define a portal window 109 when the portal is in the open position. The portal window 109 is defined by the opposing major surfaces 104a, 106a of the paddles 104, 106, and a bridge surface 170 of the base 102. The bridge surface 170 extends between the surfaces 104a, 106a and has a concave curvature (see Figure 3). As best shown in Figure 3, the side of the portal window 109 opposite the bridge surface 170 is open (i.e., unobstructed) such that the surfaces 104a, 106a and the bridge surface 170 form an inverted U-shaped end profile. In other words, the portal window 109 is defined by a channel having an open side positioned opposite the base 102.

Referring to Figure 1, the portal window 109 has a sight line 180. Pivot axes of the hinges 108, 110 are aligned generally parallel to the sight line 180. Also, the paddles 104, 106 include distraction portions 194, 196 that project outwardly from the base 102 in a direction generally parallel to the sight line 180.

Referring to Fig. 1, the base 102 has an edge 181 that faces generally toward the distraction portions 194, 196 of the paddles 104, 106. In the depicted embodiment, the edge 181 is contoured with a convex curvature that approximates the curvature of the anterior side of a vertebral body 504 against which the edge 181 is intended to abut against in use of the portal 100. For other applications, the shape of the edge 181 can be varied to correspond to the shape of other vertebral bodies or other tissue desired to be distracted. In other embodiments, the edge 181 can include at least one tooth 183 (see Fig. 5) that projects outwardly from the base 102 in the same direction as the distraction portions 194, 196. The tooth or teeth are adapted for embedding in the body against which the edge 181 abuts to assist in stabilizing the portal.

The base 102 of the portal 100 spans a spacing S (labeled in Fig. 2) defined between the two paddles 104, 106. In one embodiment, the spacing S is in the range of about 11 to about 33 millimeters. Each of the paddles 104, 106 has a height H (labeled in Fig. 4), a thickness T (labeled in Fig. 2) and a length L1 (labeled in Fig. 2). The height H corresponds to a distraction spacing desired to be provided between two adjacent vertebrae. In one embodiment, the height H is in the range of about 4 to about 18 millimeters. The thickness T is selected to allow the paddles 104, 106 to be inserted into the space between two vertebrae prior to distraction. In one embodiment, the thickness T is in the range of about 1 to about 2 millimeters. The length L 1 is the length of the distraction portion of each paddle 104, 106, and is selected so that the paddles extend a sufficient depth into the intervertebral space to provide a uniform and stable distraction spacing when the portal is opened. In one embodiment, the length L1 is in the range of about 12 to about 30 millimeters. The portal 100 also has a length L2 (labeled in Fig. 2) that can vary greatly depending on the desired type of surgery technique intended to be used. In one embodiment, the length L2 will range from about 4 to about 320 millimeters. For percutaneous or minimally invasive/closed procedures, it is desirable for the length L2 to be at the higher end of the above range. For such embodiments, the portal can form a window that extends through the soft tissue of a patient so as to provide both vertebral and soft tissue distraction during a spinal implantation procedure. For open procedures, it is desirable for the length L2 to be at the shorter end of the above range. Other dimensions than those specifically identified with respect to S, H, T, L1 and L2 above can also be used.

When the paddles 104, 106 of the portal 100 are in the closed position, the height of the paddles 104, 106 is defined by dimension T. In this orientation, the distracter portions 194, 196 of the paddles 104, 106 can easily be inserted into the intervertebral space between two adjacent vertebrae without requiring the vertebrate to be previously distracted. When the paddles 104, 106 are fully open, a distraction spacing between the vertebrae 104, 106 is set by dimension H of the paddles 104, 106. In alternative embodiments, the paddles may not be completely upright when in the open position (e.g., the paddles can be pivoted past perpendicular or short of perpendicular).

It will be appreciated that the paddles and base of the portal can be made of any number of different types of material. Example materials include stainless steel, titanium, carbon-filled polyaryletheretherketone (PEEK), plastics, composite materials as well as other materials.

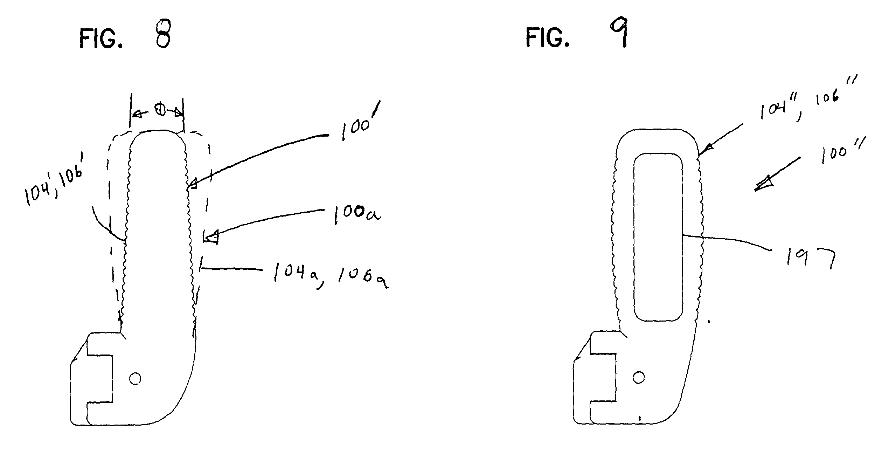

Depending on the particular application desired, different embodiments of paddles can be used. Figure 8 shows an alternative portal 100' having the same configuration as portal 100 except paddles 104', 106' have a height with a lordotic taper angle θ. In certain embodiments, this taper angle ranges from about 3 to about 8 degrees. Figure 9 shows an alternative portal 100" having the same configuration as portal 100 except paddles 104", 106" include windows 197 for improving the radiolucence of the paddles (e.g., windows allow the intervertebral space to be better viewed under fluoroscopy). Alternatively, paddles 104, 106 could be made of a radiolucent material such as carbon-filled polyaryletheretherketone (PEEK) or other suitable materials.

In other embodiments, an alternative portal 100a can be adapted for posterior insertion, and alternative paddles 104a, 106a can have a kyphotic taper angle (shown in phantom line on Fig. 8). In still other embodiments, additional pivot or hinge locations can be provided to further compact the device for insertion. For example, hinge locations can be provided along the base member for allowing the base to fold to a more compact orientation during insertion. Moreover, in still other embodiments, a plurality of paddles having different heights can be connected to the base member at each hinge location. Distraction can be accomplished by progressively pivoting the paddles open starting with the paddles of lesser height and finishing with the paddles of greater height. In still other embodiments, the paddles can include paddle portions that move telescopically relative to one another to adjust the heights of the paddles. For such embodiments, ratchet teeth can be provided for retaining the paddle portions at a desired position corresponding to a desired paddle height.

Portals in accordance with the principles of the present disclosure can be made by a number of different manufacturing techniques. One method for manufacturing the portal 100 of Fig. 1 includes first making the paddles 104, 106 and the base member 102 as three separate parts (e.g., via manufacturing techniques such as machining, casting, injection molding, etc.). After the parts have been made, the paddles 104, 106 are connected to the base member 102 by the hinge pins 108,110. Representative materials for making the parts 102, 104 and 106 of the portal typically include biocompatible materials such as titanium, stainless steel, ceramics, graphite, carbon fiber materials, and various plastics and composites of the foregoing.

### II. Example Actuator

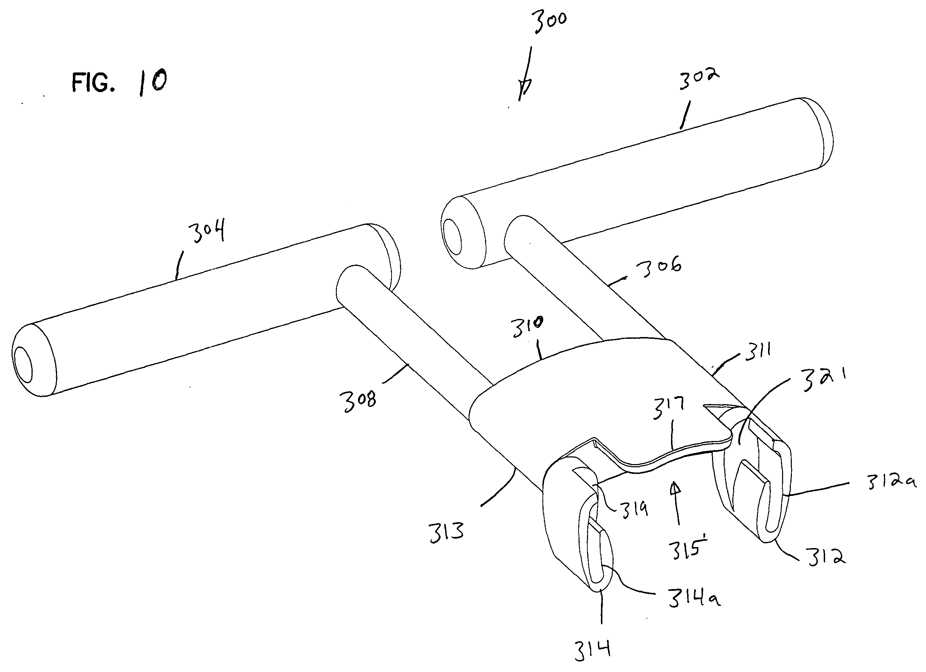

Figure 10 illustrates an exemplary actuator 300 for use in inserting the portal 100 into the intervertebral space between two adjacent vertebrae. Actuator 300 includes rotatable handles 302, 304, shafts 306, 308 coupled to the handles 302, 304, and a collar 310 that links the shafts 306, 308 together. The collar 310 includes sleeves 311, 313 in which the shafts 306, 308 are respectively mounted. The shafts 306, 308 are free to rotate within the sleeves 311, 313.

The front of the actuator 300 defines a nest 315 for receiving the portal 100. The nest 315 is defined between a top tab 317 that is part of the collar 310, and a pair of paddle receivers 312, 314 that are coupled to the ends of the shafts 306, 308. The paddle receivers 312, 314 define pockets 312a, 314a sized to receive ends 200, 202 of the paddles 104, 106.

Collar 310 connects the shafts 306,308 so that the shafts 306,308 remain an equal distance from each other. Shaft 306 is attached between rotatable handle 302 and receiver 312, and shaft 308 is attached between rotatable handle 304 and receiver 314. The shafts 306,308 are approximately parallel to each other. The receivers 312,314 are configured to move in response to movement in the rotatable handles 302,304. The rotatable handles 302,304 are provided so the receivers 312, 314 can be manipulated externally by movement of the rotatable handles 302,304. Receivers 312, 314 are shaped to receive edges 112, 120 of the paddles 104, 106 when the flip-up portal 100 is installed on the actuator 300. Upward rotational movement of the rotatable handles 302 and 304 causes flip-up portal 100 to move from the closed position to the open position. Downward rotational movement of the rotatable handles 302 and 304 causes flip-up portal 100 to move from the open position to the closed position.

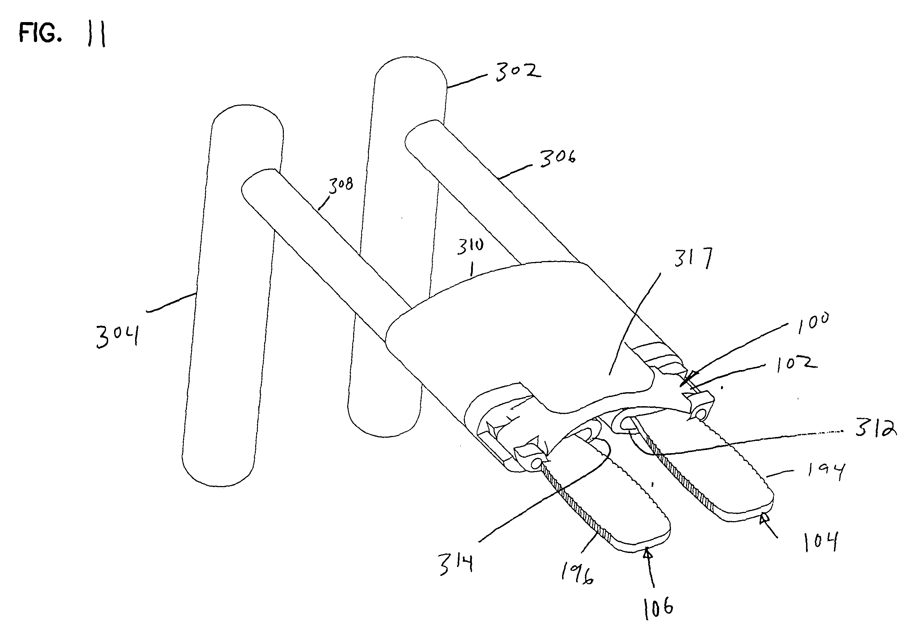

Figure 11 shows flip-up portal 100 installed on actuator 300. As installed, the edges 112, 120 of the paddles 104, 106 are inserted within the pockets 312a, 314a of the receivers 312, 314, and the base 102 is seated within the nest 315 between the tab 317 and the receivers 312, 314. When fully inserted in the pockets 312a, 314a, the ends 190, 192 of the paddles 104, 106 abut against back walls 319, 321 (visible in Fig. 10) of the receivers 312, 314.

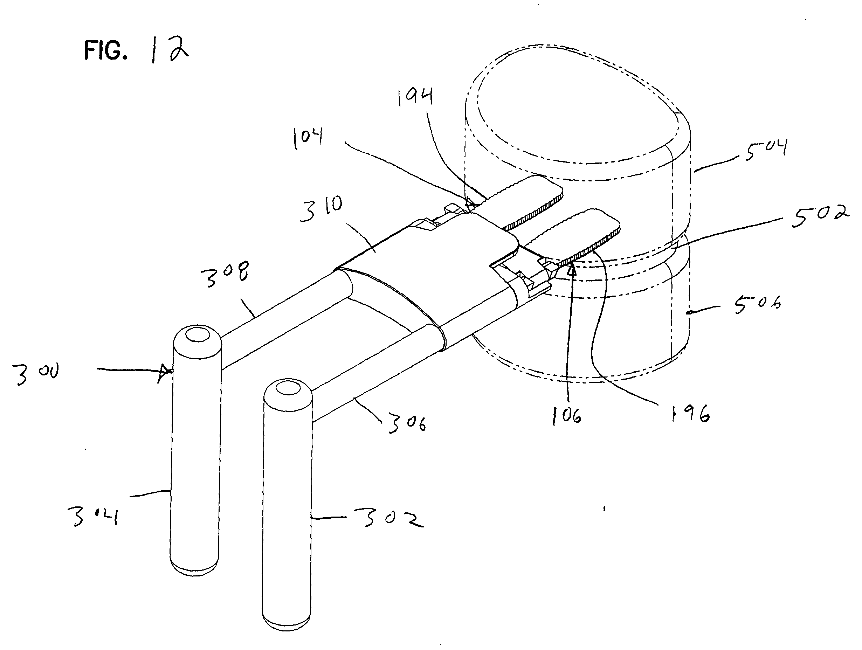

The portal 100 can be installed on the actuator 300 when the actuator handles 302, 304 are in the upwardly pivoted position of Fig. 10. After flip-up portal 100 is installed onto the actuator 300, the paddles 104, 106 are moved to the closed position (shown in Fig. 11) by pivoting the handles 302, 304 downwardly, and the actuator 300 is then used to insert the distraction portions 194, 196 of the portal 100 into disc space 502 as shown in Figure 12. Disc space 502 is the disc space in between two adjacent vertebrae 504,506 of a human spine 501. The distraction portions 194, 196 of the portal 100 are fully inserted into the intervertebral space 502 when base 102 of portal 100 abuts against vertebrae 504 (see Figure 12).

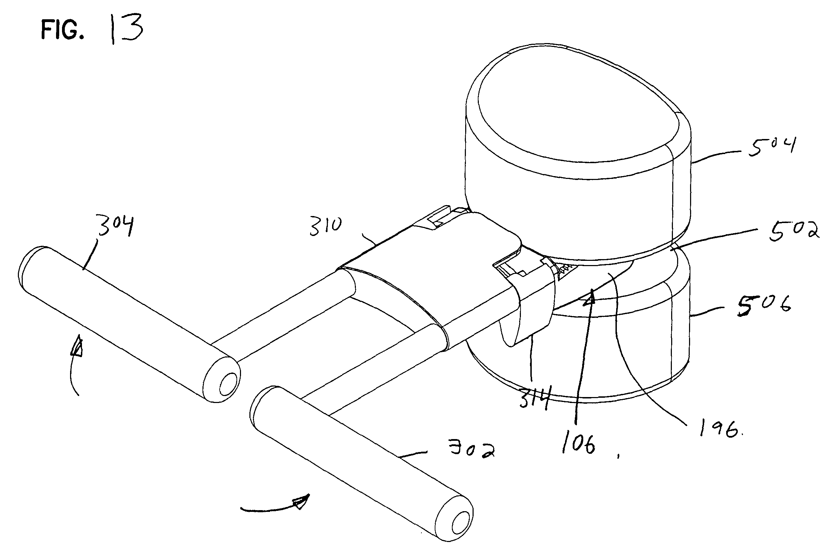

After the distraction portions 194, 196 of the paddles 104, 106 are fully inserted into disc space 502, the rotatable handles 302,304 of the actuator 300 are rotated upwardly. Upward rotation of the rotatable handles 302,304 causes the paddles 104, 106 of the portal 100 to pivoted from the closed position of Figure 13 to the open position of Figure 13. As the paddles 104, 106 pivot, the paddles 104, 106 move from a less upright orientation toward a more upright orientation. This increases the heights of the paddles thereby causing the vertebrae 504, 506 to be forced apart (i.e. distracted).

Once the paddles 104, 106 have been moved to the open position, the actuator 300 is removed from the portal 100 while leaving the distraction portions 194, 196 of the paddles 104, 106 inserted within the intervertebral space 502. With the portal 100 so inserted, the distraction portions 194, 196 of the paddles 104, 106 hold the vertebrae 504, 506 distracted, and the portal window 109 provides access to the intervertebral space 502. Thus, once the actuator 300 has been removed, one or more surgical procedures can be performed through the portal window 109. After the surgical procedures have performed, flip-up portal 100 is removed from disc space 502.

In other embodiment, a single-handled actuator could be used to concurrently pivot both paddles. Moreover, in still other embodiments, the actuator can be integral with the portal device.

### III. Example Lumbar Implantation Procedure

According to one example, a surgical procedure using flip-up portal 100 and actuator 300 proceeds as follows. First, the patient is placed in a supine position on a radiolucent table. A pad is placed under the lumbar spine to maintain or enhance lordosis. The arms are positioned 90 degrees to the torso, or folded across the chest, to maintain lordosis. General anesthesia is administered to the patient.

Next, exposure is performed as follows. The lumbar spine is exposed through a low transverse or paramedian incision, and a retroperitoneal approach is developed. The anterior rectus sheath is split longitudinally about 3-4 cm left of the midline, anterior to the level(s) to be fused. The retroperitoneal plane can be developed from an anterior direction by retracting the left rectus abdominus muscle laterally to the left, exposing the peritoneum below the arcuate line and the posterior rectus sheath above it. The peritoneum is stripped from the undersurface of the posterior rectus sheath, and the rectus sheath is divided near its insertion on the lateral wall, from inferior to superior, until sufficient exposure is achieved. The left psoas, great vessels, left ureter, and left sympathetic trunk are identified and left protected from injury.

Normally, the L5-S1 disc level can be exposed below the bifurcation of the great vessels. To expose the L4-5 level, the left iliac vein and/or vena cava is mobilized to the right lateral margin of the spine. Deep vascular dissection of the small vessels that branch off the vena cava and aorta is performed for safe implant placement. For L5-S1 implantation, the middle sacral vessels are identified, clipped, and sectioned to provide adequate exposure, and at L4-5 the segmental vessels above the disc and the ascending lumbar vein are dissected.

After complete exposure of the anterior aspect of the lumbar spine, a pin, needle, or screw is inserted approximately in the midline of the involved disc. Following midline identification, location of the vertebral body is marked, for example with a pen or small cautery mark. An anterior-posterior radiograph is taken to verify the location of the midline. A window, approximately the size of flip-up portal 100, is cut symmetrically about the midline, in the annulus and a complete discectomy is performed. The lateral annulus is retained to act as a tension band. An appropriately sized flip-up portal 100 and actuator 300 are selected based on preoperative templating. The flip-up portal 100 is then inserted into the disc space as described above. Proper positioning can be confirmed with fluoroscopy. The paddles of the flip-up portal 100 are then moved to the open position to distract the adjacent vertebrae. After the portal has been opened, the actuator 300 is removed from the portal.

Next, the endplate is prepared for anterior column support. A rasp of appropriate size is selected according to the size of flip-up portal 100. The endplate is prepared by inserting the rasp head into the intervertebral space though the portal window 109, and by rasping in an anterior/posterior direction. The endplate can also be prepared for fusion lattice by inserting a chiseling device through the portal window 109 to chisel away portions of the endplate to expose cancellous bone. Thereafter, an implant/graft can be inserted into the intervertebral space through the portal window 109. It will be appreciated that any number of different implant configurations can be used. Example techniques for inserting an implant through a portal are described in U.S. Application Serial No. 10/080,375, filed February 19, 2002 and entitled "Bone Implants And Methods", which is hereby incorporated by reference in its entirety. After insertion of the implant, the actuator 300 is used to remove flip-up portal 100 from the disc space.

It will be appreciated that portals in accordance with the present invention can be used for lumbar, thoracic or cervical spinal implantation applications, and can be used in open, percutaneous, minimally invasive, or any other suitable procedures. Further, portals in accordance with the present invention can be used in posterior approach procedures, anterior approach procedures, posterior lateral approach procedures, oblique procedures, as well as other procedures.

The invention also provides a method of distraction of a disc space between adjacent vertebrae of a spine of a patient for a surgical procedure, comprising:
inserting a flip-up portal into the disc space while the portal is in an insertion orientation; and
expanding the portal to a distraction orientation in which the portal separates the vertebrae and provides a portal window for assessing the disc space. In said method, the portal advantageously includes a bridge member and two paddles connected to the bridge member, the portal being moved from the insertion orientation to the distraction orientation by moving the paddles relative to the bridge member. Advantageously, the paddles are moved from a less upright orientation to a more upright orientation when the portal is moved from the insertion orientation to the distraction orientation. Advantageously, the paddles are generally co-planar when the portal is in the insertion orientation. Advantageously, the paddles project a first distance from the bridge member when the portal is in a first, insertion orientation and project a second distance from the bridge member when the portal is in a second, distraction orientation, the first distance being less than the second distance.

With regard to the forgoing description, it is to be understood that changes may be made in detail, especially with respect to the shape, size and arrangement of the parts. It is intended that the specification and depicted aspects be considered illustrative only and not limiting with respect to the broad underlying concepts of the present disclosure.

## Claims

1. A portal for use in a spinal implantation procedure comprising:
a base;
a first paddle that is pivotally coupled to the base; and
a second paddle that is pivotally coupled to the base, wherein the first paddle and the second paddle are dimensioned to fit in a disc space between two adjacent vertebrae of a spine of a patient.

2. The portal as claimed in claim 1, wherein the first paddle has a height in the range of 4 to 18 mm, and the second paddle has a height in the range of 4 to 18 mm.

3. The portal as claimed in claim 1 or claim 2, further comprising a first pivot hinge that connects the first paddle to the base, and a second pivot hinge that connects the second paddle to the base.

4. The portal as claimed in any one of claims 1 to 3, wherein the first paddle is coupled to one end of the base, and the second paddle is coupled to the opposite end of the base.

5. The portal as claimed in any one of claims 1 to 4, wherein the first paddle and the second paddle are connected to the base such that the first paddle and the second paddle may be pivoted between an open position and a closed position, wherein, in the closed position the first paddle and the second paddle are both approximately parallel to the base, and in the open position the first paddle and the second paddle are both approximately perpendicular to the base, such that the portal is configured to distract the two adjacent vertebrae apart when the first paddle and the second paddle are in the open position.

6. The portal as claimed in any one of claims 1 to 5, wherein the first paddle has rounded comers, and the second paddle has rounded comers.

7. The portal as claimed in any one of claims 1 to 6, wherein edges of the first paddle have surface irregularities, and edges of the second paddle have surface irregularities.

8. The portal as claimed in claim 7, wherein the surface irregularities of the first handle comprise serrations, and the surface irregularities of the second handle comprise serrations.

9. The portal as claimed in any one of the preceding claims, wherein the first paddle comprises a first lordotic paddle, and the second paddle comprises a second lordotic paddle.

10. The portal as claimed in any one of the preceding claims, wherein the first paddle comprises a first radiolucent paddle, and the second paddle comprises a second radiolucent paddle.

11. A portal for use in distracting adjacent vertebrae, the portal comprising:
first and second paddles, the paddles having heights sized to correspond to a desired distraction spacing between the adjacent vertebrae, and thicknesses sized to permit insertion of the paddles between the vertebrae prior to distraction of the adjacent vertebrae; and
a bridge member that spans a distance between the first and second paddles, the first and second paddles being moveable relative to the bridge between a closed position and an open position, wherein the paddles are adapted for insertion between the adjacent vertebrae prior to distraction when in the closed position, and the paddles are adapted to maintain the desired distraction spacing between the adjacent vertebrae when in the open position.

12. The portal as claimed in claim 11, wherein the paddles each move from a less upright position to a more upright position when pivoted from the closed position to the open position.

13. The portal as claimed in any one of the preceding claims, wherein the paddles each include major surfaces that face towards the base or bridge member when the paddles are in the closed position, and that face toward each other when the paddles are in the open position.

14. The portal as claimed in any one of the preceding claims, wherein the paddles and the base or bridge member cooperate to define a portal window when the paddles are in the open position, the portal window defining a sight line into an intervertebral space defined between the adjacent vertebrae.

15. The portal as claimed in any one of the preceding claims, wherein the paddles include distracter potions that project outwardly from the base or bridge member in a direction that extends along the sight line of the portal window.

16. The portal as claimed in any one of the preceding claims, wherein the heights of the paddles are in the range of 4-18 mm, and the thicknesses of the paddles are in the range of 1-2 mm.

17. The portal as claimed in any one of the preceding claims, wherein the paddles are generally parallel to the base or bridge member when in the closed position, and generally perpendicular to the base or bridge member when in the open position.

18. A portal for use in a spinal implantation procedure comprising:
a base having a length between a first end and an opposite second end;
a first paddle sized to fit in a disc space between two vertebrae, the first paddles having a first edge, a second edge, and a third edge, and a fourth edge, wherein the first edge is approximately parallel to the second edge, the third edge is approximately parallel to the fourth edge, and the first edge is approximately perpendicular to the third edge, wherein the first paddle has a width between the third edge and the fourth edge, and a length between the first edge and the second edge, wherein the first paddle is pivotally coupled to the first end of the base and is pivotally movable between a first position and a second position, and wherein the first paddle is approximately parallel to the base when the first paddle is in the first position, and the first paddle is approximately perpendicular to the base when the first paddle is in the second position;
a second paddle sized to fit in the disc space between two vertebrae, the second paddle having a first edge, a second edge, a third edge, and a fourth edge, wherein the first edge of the second paddle is approximately parallel to the second edge of the second paddle, the third edge of the second paddle is approximately parallel to the fourth edge of the second paddle, and the first edge of the second paddle is approximately perpendicular to the third edge of the second paddle, wherein the second paddle has a width between the third edge and the fourth edge, and a length between the first edge and the second edge, wherein the second paddle is pivotally coupled to the second end of the base, and the second paddle is pivotally movable between a first position and a second position, wherein the second paddle is approximately parallel to the base when the second paddle is in the first position, and the second paddle is approximately perpendicular to the base when the second paddle is in the second position.

19. The portal as claimed in claim 18, wherein the first paddle and the second paddle are approximately parallel to each other when the first paddle and the second paddle are in the second position.

20. The portal as claimed in any one of the preceding claims, wherein portal has a spacing of 11-33 mm between the paddles when the paddles are open, and wherein the paddles each have a height in the range of 4-18 mm, a distraction length in the range of 12-30, and a thickness in the range of 1-2 mm.

21. A spinal distractor system comprising:
a flip-up portal comprising a base, a first paddle, and a second paddle, the first paddle and the second paddle being movable between open and closed positions, the first and second paddles being approximately parallel to the base when in the closed position and the first and second paddles being approximately perpendicular to the base when in the open position; and
an actuator for insertion of the flip-up portal into a disc region between two vertebrae, the actuator including at least one handle that is rotated to move the first and second paddles from the closed position to the open position.

22. The spinal distractor system as claimed in claim 21, wherein the actuator further comprises:
a first receiver for receiving an end of the first paddle, and a first shaft that connects the first receiver to the first handle;
a second receiver for receiving an end of the second paddle, and a second shaft for connecting the second receiver to the second handle; and
a collar that couples the first and second shafts together.
